Europäisches Patentamt

European Patent Office

Office européen des brevets

(11) Numéro de publication: **0 507 035 A1**

# DEMANDE DE BREVET EUROPEEN

(21) Numéro de dépôt: **91400898.2**

(22) Date de dépôt: **03.04.91**

(51) Int. Cl.5: **A61K 7/48**, A61K 35/78

(43) Date de publication de la demande:
**07.10.92 Bulletin 92/41**

(84) Etats contractants désignés:
**AT BE CH DE DK ES FR GB GR IT LI LU NL SE**

(71) Demandeur: **SYNTHELABO**
**58 rue de la Glacière**
**F-75013 Paris(FR)**

(72) Inventeur: **Fabre, Bernard**
**4, rue du Pressoir**
**F-37270 Saint-Martin-le-Beau(FR)**
Inventeur: **Potier, Anne**
**39-41, rue Emile-Zola, Appt 10**
**F-37100 Tours(FR)**
Inventeur: **Faust, Patrick**
**12, Allée M. Pimpore**
**F-37540 Saint-Cyr-sur-Loire(FR)**

(74) Mandataire: **Thouret-Lemaitre, Elisabeth et al**
**SYNTHELABO Service Brevets 22, avenue**
**Galilée, B.P. Box 72**
**F-92352 LE PLESSIS ROBINSON CEDEX(FR)**

(54) **Extraits de Reine des Prés, leur préparation et leurs applications.**

(57) Extrait de Reine des Prés, caractérisé par le fait qu'il contient de l'acide chlorogénique, de l'acide gallique et des flavonoïdes, et par le fait que la teneur en acide gallique est de 0,2 à 8 % en poids et la teneur en flavonoïdes est de 2 à 5 % en poids, par rapport à la poudre pour un extrait sec et par rapport au résidu sec pour un extrait liquide ou mou.
Application en cosmétique.

EP 0 507 035 A1

La présente invention a pour objet l'obtention d'extraits de Reine des Prés, *Spirea ulmaria* L., les extraits ainsi obtenus et leurs applications.

La Reine des Prés, *Spirea ulmaria* L., appartient à la famille des rosacées. C'est une plante vivace, aux tiges raides de 60 à 130 cm, aux feuilles pennées, vertes en dessus, légèrement argentées en dessous. Les fleurs sont petites, blanc crème, odorantes, disposées en ombrelles corymbiformes. L'époque de la floraison s'étale de mai en août, dans les prairies et les bois humides. La Reine des Prés est commune en France. On la trouve dans presque toute l'Europe, sauf le littoral méditerranéen, en Asie et en Amérique du Nord.

La composition chimique de la plante est la suivante :
- Acides phénoliques dans les parties aériennes :
  Acides caféique, syringique, p-coumarique, protocatéchique, vanillique, chlorogénique, gallique,
- Flavonoïdes dans les parties aériennes :
  Hypéroside (quercétine-3-galactoside) Avicularine (quercétine-3-arabinofuranoside) Spiraeoside (quercétine-4'-glucoside),
- Huiles essentielles dans les parties aériennes :
  Aldéhyde salicylique
  Salicylate de méthyle
  Linalol
  Terpinéol
  Géraniol
  Trans-anéthol
  $\beta$-ionon,
- Dérivés salicylés dans les parties aériennes
  Les formes libres sont volatiles et sont donc des constituants des huiles essentielles : aldéhyde salicylique, salicylate de méthyle ;
  il existe également des formes liées, généralement des hétérosides comme le monotropitoside dont l'aglycone est le salicylate de méthyle lié à un glucose-xylose,
- Tanins dans les racines et les rhizomes.

Selon l'invention, on soumet les plantes entières de Reine des Prés sèches ou fraîches, broyées ou pulvérisées, à une extraction par un solvant tel que l'eau, un alcanol en $C_1$-$C_4$,l'acétone ou le propylèneglycol, ou un mélange de ces solvants. Par exemple, on peut utiliser un mélange alcanol $C_1$-$C_4$/eau ou un mélange acétone/eau en proportions de 96/4 à 30/70 en volume. On peut également utiliser un mélange propylèneglycol/eau dans des proportions de 100/10 à 40/60 en volume.

La quantité de solvant d'extraction utilisée est égale à 4 à 20 fois le poids de plantes entières de Reine des Prés. On effectue l'extraction éventuellement sous agitation à une température située entre 10°C et l'ébullition du solvant. La durée de l'extraction est de 2 à 80 heures. On concentre éventuellement les solutions extractives. On sèche éventuellement les concentrats obtenus, soit en étuve sous vide, soit par nébulisation, soit par lyophilisation, soit par séchage à l'aide d'un sécheur-réacteur cylindrique.

Les extraits de plantes entières de Reine des Prés obtenus selon l'invention sont caractérisés par la présence d'acides phénoliques, tels que l'acide gallique et l'acide chlorogénique, de flavonoïdes tels que l'hypéroside (quercétine-3-galactoside), l'avicularine (quercétine-3-arabinofuranoside) et le spiraeoside (quercétine-4'-glucoside), et, après saponification et hydrolyse d'une solution éthanolique (à 50 % v/v) de l'extrait de Reine des Prés selon l'invention, par la présence d'acide salicylique.

On met en évidence ces différents constituants par les tests suivants : on soumet une solution éthanolique de l'extrait de Reine des Prés obtenu selon l'invention, à la réaction à la cyanidine de Shibata (Shibata K., Nagai I., Kishida M., 1916, Journ. Biol. Chem. 28, p. 93). Cette réaction, caractéristique des flavonoïdes à l'exception des chalcones et des isoflavones, s'effectue sur une solution éthanolique (96 % v/v) de l'extrait de Reine des Prés de l'invention. Après agitation et filtration, on ajoute 3 ml d'acide chlorhydrique concentré à 5 ml du filtrat ; l'addition de tournure de magnésium provoque l'apparition d'une coloration rouge-orangé et d'un dégagement d'hydrogène caractéristique des flavonoïdes. Cette coloration s'intensifie par ajout d'alcool amylique.

On met en présence une solution éthanolique (96 % v/v) d'extrait de Reine des Prés obtenu selon l'invention et une solution de cyanure de potassium. La solution se colore en rouge-orangé, coloration caractéristique de l'acide gallique. La même solution donne ensuite un précipité marron-jaune révélant la présence de tanins.

On identifie l'acide gallique par chromatographie sur couche mince de gel de silice. On utilise une phase mobile composée d'un mélange de n-butanol, d'eau et d'acide acétique dans les proportions 4-5-1. Après migration, la révélation par une solution de chlorure ferrique à 10 % en volume dans une solution éthanolique (96 % v/v) permet d'identifier l'acide gallique recherché.

On identifie également l'hypéroside (quercétine-3-galactoside) et l'acide chlorogénique par chromatographie sur couche mince de gel de silice. On utilise une phase mobile composée d'un mélange d'acétate d'éthyle, de méthyléthylcétone, d'acide formique et d'eau dans les proportions 5-3-3-1. On pulvérise sur la plaque chromatographique une solution de diphénylborate d'aminoéthanol à 1 % p/v dans le méthanol, ce qui révèle la présence d'hypéroside et d'acide chlorogénique.

L'acide salicylique est mis en évidence par chromatographie sur couche mince, après saponification et hydrolyse d'une solution d'extrait de Reine des Prés selon l'invention : on dissout 1 g d'extrait de Reine des Prés selon l'invention dans 50 ml d'une solution aqueuse d'éthanol à 50 % v/v contenant 1 g de potasse. On porte ce mélange à ébullition sous reflux pendant 2 heures. On refroidit à température ambiante, puis on filtre la solution et on l'amène à pH 1 par addition d'acide chlorhydrique concentré. On porte cette solution acide à ébullition sous reflux pendant 1 heure, puis on la filtre et on la neutralise avec une solution sodique à 20 % v/v. On concentre à sec, puis on reprend le résidu dans de l'éthanol absolu, on le filtre et on le concentre. On dépose cette solution concentrée sur une plaque de gel de silice. On utilise une phase mobile constituée d'un mélange de benzène, de méthanol et d'acide acétique dans les proportions 45-8-3. On pulvérise sur la plaque chromatographique une solution de chlorure ferrique à 10 % dans une solution éthanolique à 96 % v/v. On révèle ainsi la présence d'acide salicylique.

On dose les flavonoïdes par spectrophotométrie. On effectue ce dosage par rapport à un témoin pur d'hypéroside. On traite l'extrait de Reine des Prés obtenu selon l'invention par du méthanol, afin d'extraire le maximum de flavonoïdes. On filtre les solutions méthanoliques, puis on les dilue dans une solution méthanolique contenant du trichlorure d'aluminium anhydre. Le trichlorure d'aluminium anhydre provoque un effet bathochrome caractéristique des flavonoïdes et permet de faire un dosage spectrophotométrique. Ce dosage s'effectue sur le maximum d'absorption UV situé à 437 nm et on l'exprime par rapport à une solution méthanolique contenant le trichlorure d'aluminium et une quantité connue d'hypéroside. Le maximum d'absorption UV de ce flavonoïde dans ce mélange se situe à 441 nm. Les teneurs en flavonoïdes varient, selon la nature de l'extrait selon l'invention, de 2 à 5 % par rapport à la poudre pour un extrait sec et par rapport au résidu sec pour un extrait liquide ou mou.

On dose l'acide gallique par chromatographie liquide haute performance. On utilise une colonne de type silice phase greffée $C_{18}$ et une phase mobile composée de proportions variables d'eau, de méthanol et d'acide acétique ; le débit est de 1 ml/mn ; la détection s'effectue à 254 nm. Les teneurs en acide gallique varient selon la nature de l'extrait obtenu selon l'invention de 0,2 à 8 % par rapport à la poudre pour un extrait sec et par rapport au résidu sec pour un extrait liquide ou mou.

Les exemples suivants illustrent l'invention :

Exemple 1.

On introduit dans un réacteur chauffant 10 kg de plantes entières de Reine des Prés préalablement broyées, ainsi que 100 litres d'eau bouillante. On laisse le mélange infuser pendant 3 heures sous agitation, puis on le filtre et on le concentre sous vide à 60°C jusqu'à ce que l'on obtienne un concentrat de 10 kg. On sèche ce dernier en étuve sous vide à 60°C, puis on le broie. On obtient ainsi une poudre de couleur brune, d'odeur faible.

Les réactions colorées d'identification des flavonoïdes, de l'acide gallique et des tanins sont positives.

On identifie l'acide gallique, l'acide chlorogénique, l'hypéroside et l'acide salicylique par chromatographie sur couche mince dans leur système chromatographique correspondant. On dose l'acide gallique par chromatographie liquide haute performance : sa teneur est de 4 % par rapport au produit sec. On dose les flavonoïdes par spectrophotométrie : leur teneur est de 2 % par rapport au produit sec.

Exemple 2.

On introduit dans un percolateur 1 kg de plantes entières de Reine de Prés préalablement broyées ainsi que 10 kg d'une solution éthanolique à 50 % v/v. On laisse macérer pendant 12 heures, puis on soutire les liqueurs pendant 24 heures. On filtre le lixiviat récupéré. On concentre le filtrat sous vide à 60°C. On nébulise alors le concentrat et on homogénéise le produit sec. Celui-ci se présente sous la forme d'une poudre de couleur brun clair, d'odeur faible.

On identifie l'acide gallique, les flavonoïdes et les tanins par leurs réactions colorées respectives. On identifie également les acides gallique, chlorogénique, salicylique ainsi que l'hypéroside par chromatographie sur couche mince.

On dose l'acide gallique par chromatographie liquide haute performance : sa teneur est de 0,5 % par rapport au produit sec. On dose également les flavonoïdes par spectrophotométrie : leur teneur est de 3,5 % par rapport au produit sec.

L'étude des extraits de Reine des Prés selon l'invention permet de mettre en évidence une activité anti-radicalaire intéressante.

On évalue cette activité par l'action des extraits selon l'invention sur un radical libre, le diphénylpicrylhydrazyl hydrate, absorbant dans le violet à 513 nm (Lamaison JL. Petitjean-Freytet C., Carnat P., Carnat A., 988, Plantes Médicinales et Phytothérapie, 22, (4), p. 231-234). Un composé anti-radicalaire, piégeur de radicaux libres, entraîne une inhibition de l'apparition de coloration du radical libre témoin, donc une diminution de la densité optique de ce radical ; l'activité anti-radicalaire d'une substance s'exprime par la diminution du pourcentage d'absorbance du radical libre testé.

Dans ces conditions de test, les extraits de Reine des Prés selon l'invention permettent d'obtenir 100 % d'atténuation à une concentration de 0,3 mg/ml, alors que les témoins, vitamine E et cynarine, donnent un pourcentage d'atténuation plus faible à une concentration plus élevée. Ces substances témoins ont été sélectionnées car, dans ces conditions de test, ce sont les substances les plus actives.

| Substances testées | Concentration | mg/ml Inhibition % |
|---|---|---|
| Vitamine E | 0,01<br>0,05<br>0,1<br>0,5<br>1 | 9<br>28<br>61<br>95<br>96 |
| Cynarine | 0,01<br>0,05<br>0,1<br>0,5<br>1 | 16<br>60<br>89<br>94<br>96 |
| Extrait aqueux de Reine de Prés caractérisé en ce que la teneur en acide gallique est de 4 % et la teneur en flavonoïdes est de 2 % par rapport au résidu sec. | 0,01<br><br>0,03<br>0,05<br>0,1<br>0,3<br>1 | 7<br><br>21<br>23<br>43<br>10O<br>100 |

On a pu également évaluer l'activité anti-radicalaire des extraits obtenus selon l'invention sur l'anion superoxyde généré par la réaction du NADH (nicotinamide adénine dinucléotide hydrate) sur le PMS (phénazine méthosulfate). L'anion superoxyde provoque la réduction du NBT (nitrobleu de tétrazolium) en diformazan, de coloration bleue intense. On dose celui-ci par spectrophotométrie (Roback J., Gryglewski RJ., Biochemical Pharmacology, 1988, 37, (5), p. 837-841). On exprime l'activité anti-radicalaire d'une substance en pourcentage d'inhibition du développement de coloration due au diformazan. Par cette méthode de dosage, les extraits de Reine des Prés selon l'invention présentent une activité supérieure à celle de deux témoins choisis : la quercétine et l'acide rosmarinique. On obtient des inhibitions de 83 % et 88 % avec des concentrations respectivement de 0,05 mg/ml et 0,1 mg/ml de l'extrait selon l'invention alors que pour obtenir un niveau d'inhibition similaire, il faut une concentration 5 à 10 fois plus élevée des témoins : 90 % pour 0,5 mg/ml d'acide rosmarinique et 91 % pour 0,5 mg/ml de quercétine. Ces substances témoins sont pourtant les substances étalons testées les plus actives dans ces conditions de test.

| Substances testées | concentrations | mg/ml inhibition % |
|---|---|---|
| Quercétine | 0,01<br>0,05<br>0,1<br>0,5<br>1 | 1<br>31<br>69<br>91<br>98 |
| Acide rosmarinique | 0,01<br>0,5<br>0,1<br>0,5<br>1 | 2<br>28<br>59<br>90<br>99 |
| Extrait aqueux de Reine des Prés caractérisé en ce que la teneur en acide gallique est de 4 % et la teneur en flavonoïdes est de 2 % en poids par rapport au résidu sec. | 0,01<br><br>0,05<br>0,1<br>1 | 52<br><br>83<br>88<br>95 |

Les extraits de Reine des Prés selon l'invention possèdent également des propriétés analgésique et antiinflammatoire dues à la présence de dérivés salicylés.

Les extraits obtenus selon l'invention sont utilisables en cosmétique, en particulier pour la protection de la peau exposée à un ensoleillement prolongé et donc agressée par les UVA et les UVB : l'activité anti-radicalaire des extraits de Reine des Prés selon l'invention permet de lutter efficacement contre les effets néfastes des UVA, qui provoquent l'apparition de radicaux libres toxiques responsables d'un vieillissement prématuré de la peau ; les activités analgésique et antiinflammatoire des extraits selon l'invention permettent de soulager l'inflammation des tissus cutanés due aux UVB.

Les extraits de plantes entières de Reine des Prés de l'invention peuvent être utilisés sous forme de pommades préparées

- soit à partir d'un extrait de Reine des Prés de l'invention, associé par exemple au mélange d'excipients monooléate de glycérol, vaseline, lanoline, cire blanche, huiles d'amande et de parrafine et eau,
- soit à partir d'un extrait de Reine des Prés de l'invention, associé par exemple au mélange d'excipients huile d'arachide, lanoline anhydre, glycérine, amidon de blé, cire blanche et eau.

Les extraits de Reine des Prés de l'invention peuvent aussi être présentés sous forme de crème dermique, lorsqu'ils sont associés à un mélange d'excipients tels que la cire d'abeille, la paraffine liquide légère, l'eau, la vaseline ou la lanoline, la cire blanche d'abeilles liquide légère, et/ou le glycérol.

Enfin, des formules de produits solaires peuvent être préparés à partir des extraits de Reine des Prés de l'invention : on peut incorporer l'extrait dans des émulsions classiques H/E (huile/eau)ou E/H (eau/huile)- ou préparer simplement des solutions huileuses d'extrait.

**Revendications**

1. Extrait de Reine des Prés, caractérisé par le fait qu'il contient de l'acide chlorogénique, de l'acide gallique et des flavonoïdes, et par le fait que la teneur en acide gallique est de 0,2 à 8 % en poids et la teneur en flavonoïdes est de 2 à 5 % en poids, par rapport à la poudre pour un extrait sec et par rapport au résidu sec pour un extrait liquide ou mou.

2. Extrait de Reine des Prés selon la revendication 1, extrait caractérisé en ce que les flavonoïdes sont l'hypéroside (quercétine-3-galactoside), l'avicularine (quercétine-3-arabinofuranoside) et la spiraeoside (quercétine-4'-glucoside).

3. Procédé d'obtention d'un extrait de Reine des Prés selon l'une quelconque des revendications 1 et 2, procédé caractérisé en ce que l'on extrait la plante entière de Reine des Prés à l'aide d'un solvant tel que l'eau, l'acétone, un alcanol en $C_1$-$C_4$, le propylèneglycol ou un mélange de ces solvants.

**4.** Procédé selon la revendication 3, procédé caractérisé en ce que l'extraction est effectuée par un mélange alcanol en $C_1$-$C_4$/ eau ou acétone/eau, mélange de 96/4 à 30/70 en volume.

**5.** Procédé selon la revendication 3, procédé caractérisé en ce que l'extraction est effectuée par un mélange propylèneglycol/eau, 100/10 à 40/60 en volume.

**6.** Procédé selon l'une quelconque des revendications 3 à 5, procédé caractérisé en ce que l'extraction est statique ou agitée.

**7.** Procédé selon l'une quelconque des revendications 3 à 6, procédé caractérisé en ce que la quantité de solvant utilisée égale 4 à 20 fois le poids de plantes entières de Reine des Prés.

**8.** Composition cosmétique, caractérisée en ce qu'elle contient un extrait de Reine des Prés selon l'une quelconque des revendications 1 et 2, en association avec tout excipient approprié.

**9.** Composition cosmétique selon la revendication 8, composition caractérisée en ce qu'elle présente une activité anti-radicalaire.

**Revendications pour les états contractants : ES, GR**

**1.** Procédé d'obtention d'un extrait de Reine des Prés, caractérisé par le fait qu'il contient de l'acide chlorogénique, de l'acide gallique et des flavonoïdes et par le fait que la teneur en acide gallique est de 0,2 à 8% en poids et la teneur en flavonoïdes est de 2 à 5% en poids, par rapport à la poudre pour un extrait sec et par rapport au résidu sec pour un extrait liquide ou mou, procédé caractérisé en ce que l'on extrait la plante entière de Reine des Prés à l'aide d'un solvant tel que l'eau, l'acétone, un alcanol en $C_1$ -$C_4$, le propylèneglycol ou un mélange de ces solvants.

**2.** Procédé selon la revendication1, caractérisé en ce que l'extraction est effectuée par un mélange alcanol en $C_1$-$C_4$/eau ou acétone/eau, mélange de 96/4 à 30/70 en volume.

**3.** Procédé selon la revendication 1, caractérisé en ce que l'extraction est effectuée par Un mélange propylèneglycol/eau, 100/10 à 40/60 en volume.

**4.** Procédé selon l'une quelconque des revendications 1 à 3, procédé caractérisé en ce que l'extraction est statique ou agitée.

**5.** Procédé selon l'une quelconque des revendications 1 à 4, procédé caractérisé en ce que la quantité de solvant utilisée égale 4 à 20 fois le poids de plantes entières de Reine des Prés.

Office européen
des brevets

**RAPPORT DE RECHERCHE EUROPEENNE**

Numero de la demande

EP 91 40 0898

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | SEIFEN-OLE-FETTE-WACHSE, vol. 108, no. 15, septembre 1982, page A 7, Augsburg, DE; "Cosmetochem" <br> * Page A 7: "Wiesengeissbart" * | 1,3-9 | A 61 K 7/48 <br> A 61 K 35/78 |
| A | FR-A-2 411 605 (CENTRE RENNES-MONTPARNASSE) <br> * En entier * | 1-9 | |
| A | STN SERVEUR DE BASES DE DONNEES, Karlsruhe, DE, Fichier Chemical Abstracts, vol. 81, no. 13, résumé no. 74942c, Columbus, Ohio, US; Z.F. SYUZEVA et al.: "Flavonoid composition of Filipendula ulmaria (queen-of-the meadow)", & NAUCH. TR., PERM. FARM. INST., 5(2), 22-6 <br> * Résumé * | 1-2 | |
| A | STN SERVEUR DE BASES DE DONNEES, Karlsruhe, DE, Fichier Chemical Abstracts, vol. 81, no. 10, résumé no. 54372p, Columbus, Ohio, US; N.N. NOVIKOVA: "Application of Filipendula ulmaria for some therapeutic preparations", & NAUCH. TR., PERM. FARM. INST., 5(2), 27-30 <br> * Résumé * | 1-9 | **DOMAINES TECHNIQUES RECHERCHES (Int. Cl.5)** <br><br> A 61 K |
| A | EP-A-0 283 349 (LAMAISON) <br> * En entier * | 1-9 | |
| A | GB-A-2 095 553 (1NTERAG RT) <br> * En entier * | 1-9 | |

Le présent rapport a été établi pour toutes les revendications

| Lieu de la recherche | Date d'achèvement de la recherche | Examinateur |
|---|---|---|
| LA HAYE | 20-09-1991 | FISCHER J.P. |

EPO FORM 1503 03.82 (P0402)